# FASCICULE DE BREVET EUROPEEN

(11) **EP 4 247 461 B1**
(45) Date de publication et mention de la délivrance du brevet: **23.10.2024**
(21) Numéro de dépôt: 21807148.8
(22) Date de dépôt: 19.11.2021
(51) Int. Cl.: A61M 15/00

(54) **EMBOUT POUR DISPOSITIF DE DISTRIBUTION DE PRODUIT**
MUNDSTÜCK FÜR EINE PRODUKTABGABEVORRICHTUNG
MOUTHPIECE FOR A PRODUCT DISPENSING DEVICE

(30) Priorité: 20.11.2020 FR 2011946
(43) Date de publication de la demande: 27.09.2023
(73) Titulaire: NEMERA LA VERPILLIERE, 38290 La Verpillière (FR)
(72) Inventeur: QUAGLIA, Benjamin, 63119 CHÂTEAUGAY (FR); GIRAUD, Myriam, 38690 Belmont (FR); VECELLIO NONE, Laurent, 37170 Chambray-lès-Tours (FR); VINCEY, Raphaël, 69003 Lyon (FR); DUMET, Clément, 73340 BELLECOMBE EN BAUGES (FR); LECLERCQ, Thomas, 69100 VILLEURBANNE (FR)
(74) Mandataire: LLR
(86) Numéro de dépôt international: PCT/EP2021/082372
(87) Numéro de publication internationale: WO 2022/106655

(56) Documents cités:
- EP-A2- 0 147 755
- EP-B1- 0 041 783
- US-A- 3 991 761
- US-A1- 2004 154 618
- US-A1- 2009 320 838
- US-A1- 2016 354 563
- US-A1- 2018 369 513
- US-A1- 2019 269 866
- US-A1- 2020 139 057

## Description

L'invention concerne le domaine des dispositifs de distribution de produit sous forme de poudre, notamment par inhalation.

On connaît déjà dans l'état la technique, notamment d'après le document EP1270034B1, un dispositif de distribution de produit par inhalation comportant un embout buccal permettant la distribution par inhalation d'un produit sous forme de poudre, durant une phase d'inspiration d'un utilisateur, par exemple pour une application rhinopharyngée ou pulmonaire. Le produit sous forme de poudre sèche se trouve initialement contenu dans une capsule, et est par exemple composé d'un produit actif et de lactose, lequel permet de conserver le produit sous forme de poudre. La capsule est disposée dans un logement, lequel communique avec l'extérieur via des trous, puis un utilisateur perfore la capsule au moyen d'aiguilles, ce qui permet au produit sous forme de poudre d'être libéré de la capsule. Une fois la capsule perforée, l'utilisateur effectue une inspiration. Un flux d'air est ainsi créé, lequel passe dans le logement, autour de la capsule pour la mettre en mouvement et expulser la poudre. Le flux d'air se mélange à la poudre générant l'aérosol de médicament. L'aérosol passe ensuite à travers l'embout et est acheminé en direction des voies respiratoires de l'utilisateur. Ainsi, le produit sous forme de poudre est distribué grâce au flux d'air créé par l'inspiration de l'utilisateur. Toutefois, dans certains cas, l'inspiration effectuée par l'utilisateur peut être peu efficace, notamment lorsque le flux d'air est trop faible.

Lorsque le flux d'air est trop faible, les particules de poudre sèche ne sont pas correctement désagglomérées. En effet, l'air joue un rôle de désagglomérant ou de dispersant des particules de poudre, que ce soit via l'interaction directe de l'air avec la poudre ou indirectement via la rotation ou le mouvement de la capsule contenant la poudre. Quand les particules ne sont pas correctement désagglomérées, elles peuvent être déposées dans une zone non souhaitée, par exemple la paroi interne de l'embout, la bouche ou la gorge, réduisant d'autant la quantité efficace de produit inhalé.

Par conséquent, la qualité de la distribution assurée par le dispositif divulgué dans ce document dépend beaucoup de la qualité de l'inspiration, de sorte qu'il ne permet pas d'assurer que le produit sous forme de poudre est toujours distribué efficacement. EP0041783 B1 et US2004/154618 A1 divulguent d'autres dispositifs de l'état de la technique. Néanmoins ces dispositifs ne divulguent pas un ratio D1/D2 entre le diamètre D1 de l'ouverture de distribution et le diamètre D2 de la grille qui est compris entre 1,5 et 5.

Pour remédier à cette défaillance, l'invention a notamment pour but de proposer un embout pour un dispositif distribution de produit sous forme de poudre, lequel permet d'assurer que le produit sous forme de poudre est distribué plus efficacement quel que soit le flux d'air généré.

A cet effet l'invention a pour objet un embout pour un dispositif de distribution de produit sous forme de poudre, comprenant :
- un conduit de distribution, comprenant une ouverture de distribution configurée pour déboucher librement dans une voie respiratoire d'un utilisateur,
- une grille disposée transversalement dans le conduit de distribution,
- une paroi pleine disposée dans le conduit de distribution autour de la grille de façon à former une restriction de distribution, la restriction de distribution présentant une section de passage inférieure à la section de passage minimale du conduit de distribution, dans lequel l'ouverture de distribution présente un diamètre D1, la grille présente un diamètre D2 et le ratio D1/D2 entre le diamètre D1 de l'ouverture de distribution et le diamètre D2 de la grille est compris entre 1,5 et 5.

Cette restriction de distribution correspond ainsi à la plus petite section de passage de tout le conduit de distribution, en d'autres termes à la plus petite section de passage d'un flux d'air traversant le conduit de distribution.

En effet, grâce à la restriction de distribution, c'est-à-dire la restriction de la section de passage du flux d'air entraînant le produit, on génère des turbulences dans le flux d'air qui s'associent à celles générées par les trous de la grille et qui améliorent la désagglomération du produit. Par conséquent, la distribution de produit est améliorée. On constate qu'en utilisant l'invention, on génère moins de variations de doses distribuées selon les utilisateurs. Une telle répétabilité est particulièrement intéressante car les inspirations peuvent être très différentes selon les utilisateurs, par exemple dans le cas d'un sportif ou d'un utilisateur présentant des difficultés respiratoires. En outre, l'invention propose ainsi de distribuer une quantité plus homogène de produit, correctement désaggloméré et peu dépendante de la qualité de l'inspiration, mais également une distribution mieux ciblée vers la zone recherchée, à savoir généralement les poumons.

On entend par section transversale ou disposition transversale, une section ou une disposition selon un plan disposé perpendiculairement à l'axe longitudinal du conduit de distribution.

Les mots transversal, longitudinal, perpendiculaire, parallèle, tangentiel ou coplanaire utilisés dans ce texte doivent se comprendre comme incluant une certaine tolérance, au moins égale à la variabilité de fabrication, par exemple de 10 degrés.

Suivant d'autres caractéristiques optionnelles de l'embout, prises seules ou en combinaison :
- Le conduit de distribution s'étend longitudinalement.
- La distribution de produit sous forme de poudre est réalisée par inhalation.
- L'espace s'étendant longitudinalement depuis la grille vers l'ouverture de distribution est libre résultant dans un conduit de distribution particulièrement simple à fabriquer, dont le démoulage est aisé.
- La grille comporte une épaisseur comprise entre 1 et 10 millimètres, de préférence voisine de 1,3 millimètres ou de 2,5 millimètres.
- La grille comporte une pluralité de trous. De préférence, la grille comporte entre 20 et 50 trous, plus préférentiellement entre 35 et 40 trous.
- Les trous sont de forme polygonale ou circulaire, de préférence de forme hexagonale.
- La grille possède une structure en nid d'abeille. On entend par structure en nid d'abeille une série de trous de section hexagonale, c'est-à-dire comportant 6 arêtes, dans la laquelle chaque arrête d'un trou est parallèle à une arête d'un trou adjacent différent et dans laquelle la distance séparant chaque trou est constante.
- La plus grande dimension transversale des trous est inférieure ou égale à l'épaisseur de la grille.
- L'embout est un embout buccal.
- La grille est disposée à une extrémité du conduit de distribution opposée à l'ouverture de distribution. Ainsi, le produit traversant la grille subit une désagglomération optimale avant que le produit ne sorte par l'ouverture de distribution.
- Le conduit de distribution comprend une section de passage constante ou croissante depuis la grille jusqu'à l'ouverture de distribution. Ainsi, le dépôt de produit dans le conduit de distribution est diminué, du fait qu'il ne présente pas une forme présentant des saillies intérieures, ce qui améliore l'efficacité de la distribution de produit.
- Le conduit de distribution comporte une section de passage de forme elliptique. Une telle section est particulièrement adaptée à la forme de la bouche de l'utilisateur.
- Le conduit de distribution présente une dimension intérieure minimale en section transversale qui est comprise entre 15 et 30 millimètres, de préférence entre 20 et 25 millimètres, plus préférentiellement est égale à environ 22,5 millimètres. Ainsi, on dispose d'un embout doté d'un diamètre intérieur relativement conséquent, permettant de ne pas transporter le produit à une vitesse trop élevée, ce qui augmente l'efficacité de la distribution de produit.
- Le conduit de distribution est tubulaire et la dimension intérieure minimale en section transversale correspond à son diamètre.
- La grille est circulaire.
- Le ratio S1/S2 entre la surface de la section de passage minimale S1 du conduit de distribution et surface de la section transversale S2 de la grille est compris entre 2 et 7. Ainsi, un tel ratio permet de générer des turbulences améliorant la désagglomération du produit.
- L'ouverture de distribution présente un diamètre D1, la grille présente un diamètre D2 et le ratio D1/D2 entre le diamètre D1 de l'ouverture de distribution et le diamètre D2 de la grille est compris de préférence entre 1,5 et 2,5, et plus préférentiellement égal à environ 1.9. Ainsi, un tel ratio permet de générer des turbulences améliorant la désagglomération du produit et la répétabilité de la distribution.
- On entend par « diamètre de l'ouverture de distribution » le diamètre du plus petit cercle inscrit à l'intérieur de l'ouverture en section transversale. On comprend que dans le cas où l'ouverture de distribution est circulaire, il s'agit de son diamètre.
- On entend par « diamètre de la grille » le diamètre du plus petit cercle entourant la grille en section transversale, par exemple le cercle circonscrit aux trous de la grille. On comprend que dans le cas où la grille est circulaire, il s'agit de son diamètre extérieur.
- La surface de la paroi pleine orientée vers l'ouverture de distribution est coplanaire avec la surface de la grille orientée vers l'ouverture de distribution.

Grâce à la coplanarité de ces surfaces, on réalise une augmentation brusque de la section de passage du flux d'air à la sortie de la grille, ce qui est différent du cas par exemple dans lequel la paroi pleine ne serait pas plane et aurait une section transversale qui augmente ou diminue progressivement vers l'ouverture de distribution en formant un entonnoir autour de la grille. La discontinuité brusque générée par la paroi plane de la paroi pleine a pour effet de maximiser la génération de turbulences dans le flux d'air, turbulences qui se combinent à celles générées par les trous de la grille puisque la grille et la paroi pleine sont dans le même plan. Cette multiplication de génération de turbulences permet d'améliorer davantage encore la désagglomération du produit, donc une distribution efficace du produit, quel que soit le flux d'air généré, en d'autres termes quelle que soit l'inspiration de l'utilisateur.
- La grille est centrée dans une section de passage du conduit de distribution. Par centrée, on entend que le plus petit cercle entourant la grille en section transversale et le plus petit cercle inscrit à l'intérieur du conduit de distribution dans cette section transversale sont concentriques. Ainsi, la distribution du produit est améliorée.
- La paroi pleine est venue de matière avec le conduit de distribution ou avec la grille ou avec le conduit de distribution et la grille. Ainsi, la fabrication est simplifiée, du fait de l'économie d'une étape d'assemblage.
- La paroi pleine est rapportée sur le conduit de distribution.
- L'embout est entièrement venu de matière. Ainsi, la fabrication est simplifiée, du fait que l'on s'affranchit de toute étape d'assemblage pour réaliser un tel embout notamment par rapport à un embout présentant un élément oscillant dans une chambre pour générer des turbulences.
- L'embout est réalisé en matériau thermoplastique, de préférence en polypropylène ou en acrylonitrile butadiène styrène.
- L'embout est en forme de U en section longitudinale, l'âme du U étant formée par la paroi pleine et la grille, et les branches du U étant formées par le conduit de distribution.

L'invention a également pour objet un dispositif de distribution par inhalation de produit sous forme de poudre comprenant un embout tel que décrit précédemment.

Suivant d'autres caractéristiques optionnelles du dispositif de distribution, prises seules ou en combinaison :
- Le dispositif de distribution comporte une chambre de dosage configurée pour contenir du produit sous forme de poudre, laquelle comprend une entrée d'air et une sortie, l'embout étant raccordé directement à la sortie de la chambre de dosage.
- Le dispositif de distribution comporte des moyens actionnables par un utilisateur et configurés pour rompre l'intégrité d'une capsule contenant du produit sous forme de poudre disposée dans la chambre de dosage.
- La capsule est disposée transversalement dans la chambre de dosage (et non longitudinalement).

### Brève description des figures

L'invention sera mieux comprise à la lecture de la description qui va suivre donnée uniquement à titre d'exemple et faite en se référant aux dessins annexés dans lesquels :
[Fig. 1] la figure 1 est une vue en perspective d'un dispositif de distribution comportant un embout selon un mode de réalisation.
[Fig. 2] la figure 2 est une vue en coupe longitudinale du dispositif de distribution représenté sur la figure 1.
[Fig. 3] la figure 3 est une vue de dessus d'une partie du dispositif de distribution représenté sur la figure 1.

### Description détaillée

Sur toutes les figures, les mêmes références se rapportent aux mêmes éléments. Les modes de réalisation suivants sont des exemples. Bien que la description se réfère à un ou plusieurs modes de réalisation, ceci ne signifie pas nécessairement que chaque référence concerne le même mode de réalisation, ou que les caractéristiques s'appliquent seulement à un seul mode de réalisation. De simples caractéristiques de différents modes de réalisation peuvent également être combinées pour fournir d'autres modes de réalisation.

Les produits, notamment pharmaceutiques, susceptibles d'être utilisés dans le dispositif de distribution sont par exemple des formulations contenant au moins un principe actif tel que les corticoïdes, les glucocorticoïdes, les peptides, les protéines, les hormones, les principes actifs d'origine biologique, les principes actifs à base de nucléotides, comme par exemple les ADN, les ARN ou les oligonucléotides, les principes actifs avec un poids moléculaire jusqu'à 1500 Da, les polysaccharides, les vaccins, les enzymes, les anticorps, les antiviraux, ou des formulations nutritionnelles ou leur mélange.

Les produits, notamment pharmaceutiques, susceptibles d'être utilisés dans le dispositif de distribution peuvent avoir pour finalité le traitement et/ou la prévention des asthmes, des bronchopneumopathies chroniques obstructives (BPCO), des maladies cardiovasculaires telles que les angines de poitrine et les crises cardiaques, des grippes notamment des grippes A et des grippes B,

Les produits, notamment pharmaceutiques, susceptibles d'être utilisés dans le dispositif de distribution sont par exemple des formulations contenant au moins une substance active tel que le salbutamol, le fluticasone, le salmétérol, le budésonide, le formotérol, le tiotropium, la béclometasone, le vilanterol, le laninamivir, le levosalbutamol, l'ipratropium, le fénotérol, leurs sels, hydrates, solvates, stéréoisomères et/ou dérivés tels que par exemple le propionate de fluticasone, le bromure de tiotropium, le dipropionate de béclometasone, le furoate de fluticasone, le propionate de fluticasone ou le bromure d'ipratropium. Les formulations peuvent également contenir une combinaison de deux ou plus substances actives comme par exemple le fluticasone avec le salmétérol, le budésonide avec le formotérol, le fluorate de fluticasone avec le vilanterol, la béclometasone avec le levosalbutamol, le fluticasone avec le formotérol, le bromure d'ipratropium avec le salbutamol ou le bromure d'ipratropium avec le levosalbutamol.

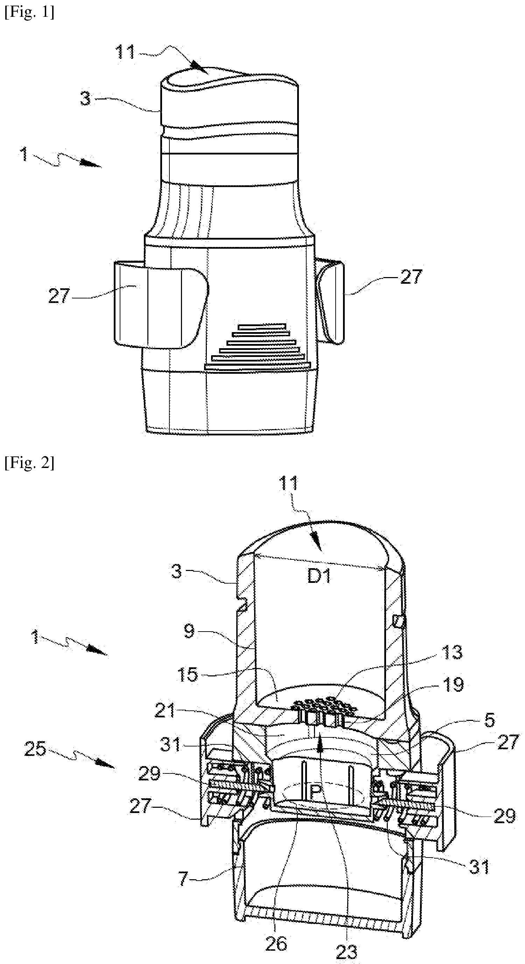

On a représenté sur la figure 1, désigné par la référence générale 1, un dispositif de distribution de produit par inhalation sous forme de poudre.

Comme représenté sur les figures 1, 2 et 3, le dispositif de distribution de produit 1 comprend un embout 3, une chambre de dosage 5 et une chambre de stockage 7.

L'embout 3 est dans cet exemple un embout buccal. Il comprend un conduit de distribution 9 s'étendant longitudinalement. Le conduit de distribution 9 comprend une ouverture de distribution 11 configurée pour déboucher librement dans une voie respiratoire d'un utilisateur, par exemple la bouche. L'embout 3 comprend également une grille 13 disposée transversalement dans le conduit de distribution 9. L'embout 3 comprend en outre une paroi pleine 15 disposée dans le conduit de distribution 9 autour de la grille 13 de façon à former une restriction de distribution 17, la restriction de distribution 17 présentant une section de passage inférieure à la section de passage minimale du conduit de distribution 9. L'espace s'étendant longitudinalement depuis la grille 13 jusqu'à l'ouverture de distribution 11 est libre. Dans cet exemple, l'embout 3 est entièrement venu de matière. L'embout 3 est réalisé en matériau thermoplastique. Comme visible sur la figure 2, l'embout 3 est en forme U en section longitudinale, l'âme du U étant formée par la paroi pleine 15 et la grille 13, et les branches du U étant formées par le conduit de distribution 9.

Le conduit de distribution 9 comprend une section de passage constante ou croissante depuis la grille 13 jusqu'à l'ouverture de distribution 11. Dans cet exemple, le conduit de distribution 9 comprend une section de passage constante depuis la grille 13 jusqu'à l'ouverture de distribution 11. Selon une variante non représentée, le conduit de distribution comprend une section de passage de forme elliptique.

Le conduit de distribution 9 présente une dimension intérieure minimale en section transversale qui est comprise entre 15 et 30 millimètres, de préférence entre 20 et 25 millimètres, plus préférentiellement est égale à environ 22,5 millimètres. Plus précisément dans cet exemple, le conduit de distribution 9 est tubulaire et la dimension intérieure minimale en section transversale correspond à son diamètre D1.

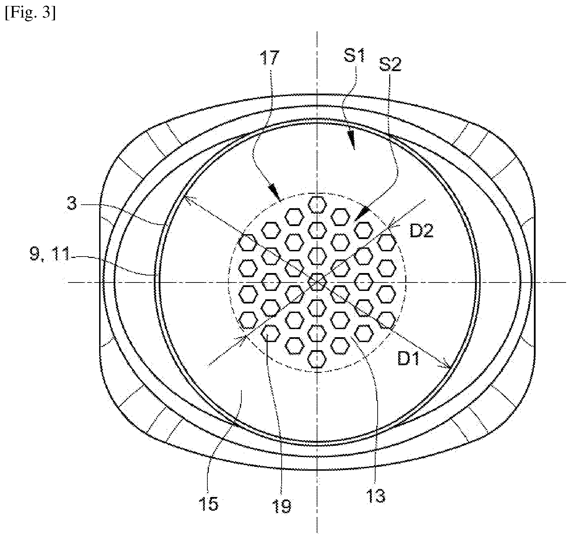

Comme représenté sur la figure 2, la grille 13 est disposée à une extrémité du conduit de distribution 9 opposée à l'ouverture de distribution 11. La grille 13 est centrée dans une section de passage du conduit de distribution 9. La grille 13 a une épaisseur comprise entre 1 et 10 millimètres, de préférence voisine de 1,3 millimètres ou de 2,5 millimètres. La grille comprend également une pluralité de trous 19. De préférence, la grille 13 comporte entre 20 et 50 trous, plus préférentiellement entre 35 et 40 trous. Dans cet exemple, la grille 13 comporte 37 trous. Les trous 19 sont de forme polygonale ou circulaire, de préférence de forme hexagonale. La grille 13 est circulaire et possède une structure en nid d'abeille, visible en particulier sur les figures 2 et 3. La plus grande dimension transversale des trous 19 est dans cet exemple égale à 1,34 millimètre, inférieure ou égale à l'épaisseur de la grille qui est de 2,5 millimètres dans cet exemple. Ainsi, les trous 19 sont formés en tant que trous longitudinaux. Le ratio S1/S2 entre la surface de la section de passage minimale S1 du conduit de distribution 9 et la surface de la section transversale S2 de la grille 13 est compris entre 2 et 7. L'ouverture de distribution 11 présente un diamètre D1 égal à 22,5 millimètres, la grille présente un diamètre D2 égal à 11,6 millimètres et le ratio D1/D2 entre le diamètre D1 de l'ouverture de distribution et le diamètre D2 de la grille est égal à 1.9 dans cet exemple.

La paroi pleine 15 est venue de matière avec le conduit de distribution 9 et avec la grille 13. Comme représenté sur la figure 2, la surface de la paroi pleine 15 orientée vers l'ouverture de distribution 11 est coplanaire avec la surface de la grille 13 orientée vers l'ouverture de distribution 11. Un tel embout 3 est par exemple obtenu par moulage par injection. Alternativement, selon une variante non représentée, la paroi pleine est rapportée sur le conduit de distribution, par exemple encliquetée dans le conduit de distribution.

La chambre de dosage 5 est configurée pour contenir du produit sous forme de poudre, laquelle comprend une entrée d'air 21 et une sortie 23, l'embout 3 étant raccordé directement à la sortie 23 de la chambre de dosage 5. Par exemple, l'embout 3 est fixé sur la chambre de dosage 5 par encliquetage. L'entrée d'air 21 est disposée tangentiellement dans la chambre de dosage 5, la sortie 23 étant disposée longitudinalement du côté de l'embout 3. Ainsi, un flux d'air entrant via l'entrée d'air 21 est tourbillonnant dans la chambre de dosage 5.

Le dispositif de distribution 1 comprend en outre des moyens 25 actionnables par un utilisateur et configurés pour rompre l'intégrité d'une capsule 26 contenant du produit P sous forme de poudre disposée dans la chambre de dosage 5. La capsule 26 est disposée transversalement dans la chambre de dosage 5. Ainsi, la capsule 26 n'est pas disposée longitudinalement dans la chambre de dosage 5. Dans cet exemple, les moyens 25 sont des moyens de perforation comprenant deux boutons coulissants 27 disposés de part et d'autre de la chambre de dosage 5, supportant chacun au moins une aiguille 29 configurée pour perforer la capsule 26 et libérer le produit P sous forme de poudre lorsqu'un flux d'air passe dans la chambre de dosage 5. Chaque bouton coulissant 27 comporte par exemple un élément de rappel, comme un ressort 31. Ainsi, chaque élément de rappel est configuré pour rappeler le bouton coulissant 27 respectif depuis une position de perforation dans une position de repos.

Alternativement, selon une variante non représentée, les moyens de performation sont des pointes.

La chambre de stockage 7 présente une forme de récipient. La chambre de stockage 7 est configurée pour le stockage d'une pluralité de capsules de produit en poudre. Comme représenté sur les figures 1 et 2, la chambre de stockage 7 est encliquetée de manière amovible avec la chambre de dosage 5.

Dans cet exemple, il a été constaté que la variabilité de la dose de produit distribuée efficacement en fonction du flux d'air généré est nettement plus faible comparée à celle d'un dispositif de distribution commercial existant du type précité. Ainsi, pour un flux d'air compris entre 30 L/min et 100 L/min, la variabilité de la dose de produit désaggloméré sous forme de particules de taille inférieure à 5 µm et distribué efficacement dans les poumons d'un utilisateur est de l'ordre de 15,6% alors que le dispositif commercial présente une variabilité dans les mêmes conditions de 28%.

L'invention n'est pas limitée aux modes de réalisation présentés et d'autres modes de réalisation apparaîtront clairement à l'homme du métier. La forme extérieure de l'embout 3 peut notamment être quelconque, du fait que cette forme extérieure n'a pas d'influence sur le flux d'air passant à l'intérieur de l'embout 3, dans le conduit de distribution 9.

### Liste de références

- 1 :: dispositif de distribution
- 3 :: embout
- 5 :: chambre de dosage
- 7 :: chambre de stockage
- 9 :: conduit de distribution
- 11 :: ouverture de distribution
- 13 :: grille
- 15 :: paroi pleine
- 17 :: étranglement de distribution
- 19 :: trou
- 21 :: entrée d'air
- 23 :: sortie
- 25 :: moyens
- 26 :: capsule
- 27 :: bouton coulissant
- 29 :: aiguille
- 31 :: ressort

- D1 :: diamètre de l'ouverture de distribution 11
- D2 :: diamètre de la grille 13
- S1 :: section de passage minimale du conduit de distribution 9
- S2 :: section transversale de la grille 13

- P :: produit

## Revendications

1. Embout (3) pour un dispositif de distribution (1) de produit (P) sous forme de poudre, comprenant :
- un conduit de distribution (9), comprenant une ouverture de distribution (11) configurée pour déboucher librement dans une voie respiratoire d'un utilisateur,
- une grille (13) disposée transversalement dans le conduit de distribution (9),
- une paroi pleine (15) disposée dans le conduit de distribution (9) autour de la grille (13) de façon à former une restriction de distribution (17), la restriction de distribution (17) présentant une section de passage inférieure à la section de passage minimale du conduit de distribution (9)
dans lequel l'ouverture de distribution (11) présente un diamètre D1, la grille (13) présente un diamètre D2 et **caractérisé en ce que** le ratio D1/D2 entre le diamètre D1 de l'ouverture de distribution (11) et le diamètre D2 de la grille (13) est compris entre 1,5 et 5.

2. Embout (3) selon la revendication précédente, lequel est un embout buccal.

3. Embout (3) selon l'une quelconque des revendications précédentes, dans lequel la grille (13) est disposée à une extrémité du conduit de distribution (9) opposée à l'ouverture de distribution (11).

4. Embout (3) selon l'une quelconque des revendications précédentes, dans lequel le conduit de distribution (9) comprend une section de passage constante ou croissante depuis la grille (13) jusqu'à l'ouverture de distribution (11).

5. Embout (3) selon l'une quelconque des revendications précédentes, dans lequel le conduit de distribution (9) présente une dimension intérieure minimale en section transversale qui est comprise entre 15 et 30 millimètres, de préférence entre 20 et 25 millimètres, plus préférentiellement est égale à environ 22,5 millimètres.

6. Embout (3) selon l'une quelconque des revendications précédentes, dans lequel le ratio S1/S2 entre la surface de la section de passage minimale S1 du conduit de distribution (9) et la surface de la section transversale S2 de la grille (13) est compris entre 2 et 7.

7. Embout (3) selon l'une quelconque des revendications précédentes, dans lequel le ratio D1/D2 entre le diamètre D1 de l'ouverture de distribution (11) et le diamètre D2 de la grille (13) est compris entre 1,5 et 2,5, et plus préférentiellement égal à environ 1.9.

8. Embout (3) selon l'une quelconque des revendications précédentes, dans lequel la surface de la paroi pleine (15) orientée vers l'ouverture de distribution (11) est coplanaire avec la surface de la grille (13) orientée vers l'ouverture de distribution (11).

9. Embout (3) selon l'une quelconque des revendications précédentes, dans lequel la grille (13) est centrée dans une section de passage du conduit de distribution (9).

10. Embout (3) selon l'une quelconque des revendications précédentes, dans lequel la paroi pleine (15) est venue de matière avec le conduit de distribution (9) ou avec la grille (13) ou avec le conduit de distribution (9) et la grille (13).

11. Embout (3) selon la revendication précédente, lequel est entièrement venu de matière.

12. Dispositif de distribution (1) par inhalation de produit (P) sous forme de poudre comprenant un embout (3) selon l'une quelconque des revendications précédentes.

13. Dispositif de distribution (1) selon la revendication précédente, lequel comporte une chambre de dosage (5) configurée pour contenir du produit (P) sous forme de poudre, laquelle comprend une entrée d'air (21) et une sortie (23), l'embout (3) étant raccordé directement à la sortie (23) de la chambre de dosage (5).

## Patentansprüche

1. Endstück (3) für eine Vorrichtung (1) zur Abgabe eines Produkts (P) in Pulverform, aufweisend:
- einen Abgabekanal (9), der eine Abgabeöffnung (11) aufweist, die so konfiguriert ist, dass sie frei in einen Atemweg eines Benutzers mündet,
- ein Gitter (13), das quer in dem Abgabekanal (9) angeordnet ist,
- eine feste Wand (15), die in dem Abgabekanal (9) um das Gitter (13) herum angeordnet ist, um eine Abgabebeschränkung (17) zu bilden, wobei die Abgabebeschränkung (17) einen Durchlassquerschnitt aufweist, der kleiner ist als der minimale Durchlassquerschnitt des Abgabekanals (9), wobei die Abgabeöffnung (11) einen Durchmesser D1 aufweist, das Gitter (13) einen Durchmesser D2 aufweist und **dadurch gekennzeichnet ist, dass** das Verhältnis D1/D2 zwischen dem Durchmesser D1 der Abgabeöffnung (11) und dem Durchmesser D2 des Gitters (13) zwischen 1,5 und 5 liegt.

2. Endstück (3) nach dem vorhergehenden Anspruch, wobei dieses ein Mundstück ist.

3. Endstück (3) nach einem der vorhergehenden Ansprüche, wobei das Gitter (13) an einem Ende des Abgabekanals (9) gegenüber der Abgabeöffnung (11) angeordnet ist.

4. Endstück (3) nach einem der vorhergehenden Ansprüche, wobei der Abgabekanal (9) einen konstanten oder zunehmenden Durchgangsquerschnitt von dem Gitter (13) zu der Abgabeöffnung (11) aufweist.

5. Endstück (3) nach einem der vorhergehenden Ansprüche, wobei der Abgabekanal (9) eine minimale innere Querschnittsabmessung aufweist, die zwischen 15 und 30 Millimetern, vorzugsweise zwischen 20 und 25 Millimetern, besonders bevorzugt etwa 22,5 Millimetern, liegt.

6. Endstück (3) nach einem der vorhergehenden Ansprüche, wobei das Verhältnis S1/S2 zwischen der Fläche des minimalen Durchgangsquerschnitts S1 des Abgabekanals (9) und der Fläche des Querschnitts S2 des Gitters (13) zwischen 2 und 7 liegt.

7. Endstück (3) nach einem der vorhergehenden Ansprüche, wobei das Verhältnis D1/D2 zwischen dem Durchmesser D1 der Abgabeöffnung (11) und dem Durchmesser D2 des Gitters (13) zwischen 1,5 und 2,5 und besonders bevorzugt bei etwa 1,9 liegt.

8. Endstück (3) nach einem der vorhergehenden Ansprüche, wobei die Oberfläche der festen Wand (15), die der Abgabeöffnung (11) zugewandt ist, mit der Oberfläche des Gitters (13), das der Abgabeöffnung (11) zugewandt ist, koplanar ist.

9. Endstück (3) nach einem der vorhergehenden Ansprüche, wobei das Gitter (13) in einem Durchgangsabschnitt des Abgabekanals (9) zentriert ist.

10. Endstück (3) nach einem der vorhergehenden Ansprüche, wobei die massive Wand (15) einstückig mit dem Abgabekanal (9) oder mit dem Gitter (13) oder mit dem Abgabekanal (9) und dem Gitter (13) ausgebildet ist.

11. Endstück (3) nach dem vorhergehenden Anspruch, das vollständig aus Material besteht.

12. Vorrichtung (1) zur Abgabe eines Produkts (P) in Pulverform durch Inhalation, die ein Endstück (3) nach einem der vorhergehenden Ansprüche aufweist.

13. Abgabevorrichtung (1) nach dem vorhergehenden Anspruch, welche eine Dosierkammer (5) aufweist, die so konfiguriert ist, dass sie ein Produkt (P) in Pulverform enthält, welche einen Lufteinlass (21) und einen Auslass (23) aufweist, wobei das Endstück (3) direkt mit dem Auslass (23) der Dosierkammer (5) verbunden ist.

## Claims

1. Nozzle (3) for a dispensing device (1) for a product (P) under powder form, comprising:
- a dispensing duct (9), comprising a dispensing opening (11) configured to open freely into a user's respiratory airway,
- a grid (13) arranged transversely in the dispensing duct (9),
- a solid wall (15) arranged in the dispensing duct (9) around the grid (13) so as to form a dispensing restriction (17), the dispensing restriction (17) having a passage cross-section smaller than the minimum passage cross-section of the dispensing duct (9)
wherein the dispensing opening (11) has a diameter D1, the grid (13) has a diameter D2, **characterised in that** the D1/D2 ratio between the diameter D1 of the dispensing opening (11) and the diameter D2 of the grid (13) is comprised between 1,5 and 5.

2. Nozzle (3) according to the preceding claim, which is a mouthpiece.

3. Nozzle (3) according to any one of the preceding claims, wherein the grid (13) is disposed at an end of the dispensing duct (9) opposite the dispensing opening (11).

4. Nozzle (3) according to any one of the preceding claims, wherein the dispensing duct (9) comprises a constant or an increasing passage cross-section from the grid (13) to the dispensing opening (11).

5. Nozzle (3) according to any one of the preceding claims, wherein the dispensing duct (9) has a minimum inner cross-sectional dimension of between 15 and 30 millimetres, preferably between 20 and 25 millimetres, more preferably equal to about 22.5 millimetres.

6. Nozzle (3) according to any one of the preceding claims, wherein the S1/S2 ratio between the minimum passage cross-sectional area S1 of the dispensing duct (9) and the cross-sectional area S2 of the grid (13) is between 2 and 7.

7. Nozzle (3) according to any one of the preceding claims, wherein the D1/D2 ratio between the diameter D1 of the dispensing opening (11) and the diameter D2 of the grid (13) is between 1.5 and 2.5, and more preferably equal to about 1.9.

8. Nozzle (3) according to any one of the preceding claims, wherein the surface of the solid wall (15) facing the dispensing opening (11) is coplanar with the surface of the grid (13) facing the dispensing opening (11).

9. Nozzle (3) according to any one of the preceding claims, wherein the grid (13) is centered in a passage section of the dispensing duct (9).

10. Nozzle (3) according to any one of the preceding claims, wherein the solid wall (15) is integral with the dispensing duct (9) or with the grid (13) or with the dispensing duct (9) and the grid (13).

11. Nozzle (3) according to the preceding claim, which is completely integral.

12. Dispensing device (1) for dispensing a product (P) in powder form by inhalation, comprising a nozzle (3) according to any one of the preceding claims.

13. Dispensing device (1) according to the preceding claim, which comprises a dosing chamber (5) configured to contain a product (P) in powder form, which comprises an air inlet (21) and an outlet (23), the nozzle (3) being connected directly to the outlet (23) of the dosing chamber (5).
